# EUROPEAN PATENT APPLICATION

(11) **EP 4 843 993 A1**
(43) Date of publication of application: **25.11.2026**
(21) Application number: 25742004.2
(22) Date of filing: 17.01.2025
(51) Int. Cl.: C07K 1/06, C07C 229/24, C07C 271/22

(54) **METHOD FOR PRODUCING PEPTIDE, COMPOUND, AND PEPTIDE SYNTHESIS REAGENT**

(30) Priority: 17.01.2024 JP 2024004954
(71) Applicant: FUJIFILM Corporation, Tokyo 106-8620 (JP)
(72) Inventor: TANAKA, Hiroaki, Ashigarakami-gun, Kanagawa 258-8577 (JP)
(74) Representative: HGF
(86) International application number: PCT/JP2025/001249
(87) International publication number: WO 2025/154779

(57) **Abstract**

An object of the present invention is to provide a method for producing a peptide capable of sufficiently suppressing aspartimide/glutarimide formation, a novel compound exhibiting a high suppressive effect on aspartimide/glutarimide formation, and a peptide synthesis reagent capable of sufficiently suppressing aspartimide/glutarimide formation. According to the present invention, there is provided a method for producing a peptide, including a step of reacting a compound represented by Formula (1) or Formula (2) with an amino terminus of an amino acid or a peptide.

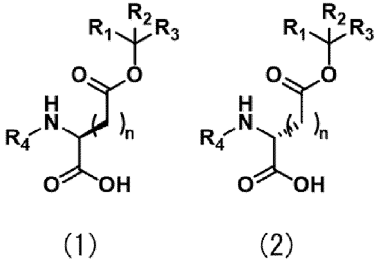

In Formula (1) and Formula (2), n is 1 or 2, R₁, R₂, and R₃ are each independently an aliphatic hydrocarbon group which may have a substituent containing no aromatic ring, at least one of R₁, R₂, and R₃ has a branched structure, two groups of R₁, R₂, and R₃ may form a ring, and R₄ represents a protecting group of an amino group.

## Description

### Technical Field

The present disclosure relates to a method for producing a peptide, a compound, and a peptide synthesis reagent.

### Background Art

Examples of the method for producing a peptide include a solid-phase method and a liquid-phase method. In the liquid-phase method, reactivity is favorable, and, after the condensation reaction, an intermediate peptide can be purified by extraction washing, isolation, or the like.

In the production of a peptide, combinations of aspartic acid or glutamic acid with another amino acid (Asp/Glu-X) are known to form aspartimide/glutarimide through an intramolecular dehydration reaction. In a case of synthesizing a peptide containing aspartic acid/glutamic acid, it has been reported that purification is complicated or the target product cannot be obtained due to the presence, as an impurity, of a peptide in which those residues have been dehydrated. For example, Non-Patent Document 1 reports that, in synthesis of a peptide containing aspartic acid, formation of aspartimide reduces the yield of the desired peptide.

Patent Document 1 discloses that an aspartic acid/glutamic acid derivative having a chain side-chain protecting group can suppress aspartimide/glutarimide formation.

### Prior Art Documents

### Non-Patent Documents

Non-Patent Document 1: RSC Chem. Biol., 2023, 4, 292-299

### Patent Documents

Patent Document 1: EP2886531B

### Summary of Invention

### Object to be solved by the invention

It has been found that the method described in EP2886531B provides an insufficient suppressive effect on aspartimide/glutarimide formation.

An object to be solved by one embodiment of the present disclosure is to provide a method for producing a peptide capable of sufficiently suppressing aspartimide/glutarimide formation.

In addition, an object to be solved by another embodiment of the present disclosure is to provide a novel compound exhibiting a high suppressive effect on aspartimide/glutarimide formation.

In addition, an object to be solved by still another embodiment of the present disclosure is to provide a peptide synthesis reagent capable of sufficiently suppressing aspartimide/glutarimide formation.

### Means for solving the object

As a result of intensive studies conducted to solve the above-described objects, the present inventors have found that introducing a branched structure into a protecting group for an aspartic acid side chain provides a high suppressive effect on aspartimide/glutarimide formation, thereby completing the present invention. According to the present disclosure, the following aspects are provided.
<1> A method for producing a peptide, comprising:
   a step of reacting a compound represented by Formula (1) or Formula (2) with an amino terminus of an amino acid or a peptide, 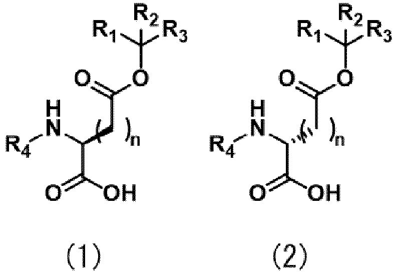
   in Formula (1) and Formula (2), n is 1 or 2, R₁, R₂, and R₃ are each independently an aliphatic hydrocarbon group which may have a substituent containing no aromatic ring, at least one of R₁, R₂, and R₃ has a branched structure, two groups of R₁, R₂, and R₃ may form a ring, and R₄ represents a protecting group of an amino group.
<2> The method for producing a peptide according to <1>, in which the compound represented by Formula (1) or Formula (2) is an N-terminal-protected amino acid or an N-terminal-protected peptide, and
   the method further comprises
   a peptide chain extension step of condensing the compound represented by Formula (1) or Formula (2) with a C-terminal-protected amino acid or a C-terminal-protected peptide; and
   a precipitation step of precipitating an N-terminal and C-terminal-protected peptide obtained in the peptide chain extension step.
<3> The method for producing a peptide according to <2>, further comprising, after the precipitation step, the following steps one or more times in this order:
   a step of deprotecting an N-terminal of the obtained N-terminal and C-terminal-protected peptide;
   a step of condensing the N-terminal-protected amino acid or the N-terminal-protected peptide with the N-terminal of the obtained C-terminal-protected peptide; and
   a step of precipitating the obtained N-terminal and C-terminal-protected peptide.
<4> The method for producing a peptide according to <2>, further comprising:
   a C-terminal deprotection step of deprotecting a C-terminal protecting group,
   in which the deprotection is performed using a trifluoroacetic acid solution of 10% by volume or less.
<5> The method for producing a peptide according to any one of <2> to <4>,
   in which a C-terminal protecting group of the C-terminal-protected amino acid or the C-terminal-protected peptide has an aliphatic hydrocarbon group having 12 or more carbon atoms.
<6> The method for producing a peptide according to any one of <1> to <5>,
   in which the aliphatic hydrocarbon group which may have a substituent containing no aromatic ring is an aliphatic hydrocarbon group which may contain -O-.
<7> The method for producing a peptide according to any one of <1> to <6>,
   in which the substituent containing no aromatic ring is a cycloaliphatic hydrocarbon group, a cycloaliphatic hydrocarbon group containing -O- in a ring, or an alkoxy group.
<8> The method for producing a peptide according to any one of <1> to <7>,
   in which the substituent containing no aromatic ring is cyclohexyl, tetrahydropyranyl, or alkoxy having 1 to 6 carbon atoms.
<9> The method for producing a peptide according to any one of <1> to <8>,
   in which R₄ is 9-fluorenylmethyloxycarbonyl or benzyloxycarbonyl.
<10> The method for producing a peptide according to any one of <1> to <9>,
   in which at least one of R₁, R₂, and R₃ is a butyl group having the branched structure, which may have the substituent containing no aromatic ring.
<11> The method for producing a peptide according to any one of <1> to <10>,
   in which n is 1.
<12> A compound represented by Formula (1) or Formula (2), 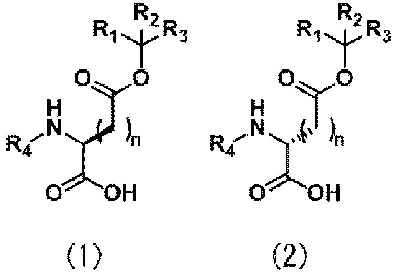 in Formula (1) and Formula (2), n is 1 or 2, R₁, R₂, and R₃ are each independently an aliphatic hydrocarbon group which may have a substituent containing no aromatic ring, at least one of R₁, R₂, and R₃ has a branched structure, two groups of R₁, R₂, and R₃ may form a ring, and R₄ represents a protecting group of an amino group.
<13> The compound according to <12>,
   in which the aliphatic hydrocarbon group which may have the substituent containing no aromatic ring is an aliphatic hydrocarbon group which may contain -O-.
<14> The compound according to <12> or <13>,
   in which the substituent containing no aromatic ring is a cycloaliphatic hydrocarbon group, a cycloaliphatic hydrocarbon group containing -O- in a ring, or an alkoxy group.
<15> The compound according to any one of <12> to <14>,
   in which the substituent containing no aromatic ring is cyclohexyl, tetrahydropyranyl, or alkoxy having 1 to 6 carbon atoms.
<16> The compound according to any one of <12> to <15>,
   in which R₄ is 9-fluorenylmethyloxycarbonyl or benzyloxycarbonyl.
<17> The compound according to any one of <12> to <16>,
   in which at least one of R₁, R₂, and R₃ is a butyl group having the branched structure, which may have the substituent containing no aromatic ring.
<18> The compound according to any one of <12> to <17>,
   in which n is 1.
<19> A peptide synthesis reagent comprising:
   the compound according to any one of <12> to <18>.

### Effect of the invention

According to one embodiment of the present invention, it is possible to provide a method for producing a peptide capable of sufficiently suppressing aspartimide/glutarimide formation.

In addition, according to another embodiment of the present invention, it is possible to provide a novel compound exhibiting a high suppressive effect on aspartimide/glutarimide formation.

In addition, according to still another embodiment of the present invention, it is possible to provide a peptide synthesis reagent capable of sufficiently suppressing the aspartimide/glutarimide formation.

### Embodiments for carrying out the invention

Hereinafter, the contents of the present disclosure will be described in detail. Descriptions of the configuration requirements set forth below may be given based on representative embodiments of the present disclosure, but the present disclosure is not limited to such embodiments.

In the present specification, unless otherwise specified, each term has the following meaning.

A numerical range expressed using "to" means a range that includes the numerical values described before and after "to" as the lower limit value and the upper limit value.

In the numerical ranges described stepwise in the present specification, the upper limit value or lower limit value recited for one numerical range may be replaced with the upper limit value or lower limit value of another numerical range described stepwise. Furthermore, in the numerical ranges described in the present specification, the upper limit value or lower limit value of the numerical range may be replaced with a value shown in the Examples.

The term "step" includes not only an independent step, but also a step that cannot be clearly distinguished from another step, so long as the intended purpose of the step is achieved.

In the notation of a group (atomic group), a notation in which substituted and unsubstituted forms are not specified encompasses both a form having no substituent and a form having a substituent. For example, the term "alkyl group" encompasses not only an alkyl group having no substituent (unsubstituted alkyl group) but also an alkyl group having a substituent (substituted alkyl group).

A chemical structural formula may in some cases be described as a simplified structural formula with hydrogen atoms omitted.

A combination of two or more preferred aspects is a more preferred aspect.

In a case where the amino acid and peptide according to the present disclosure have a hydroxy group, an amino group, a carboxy group, a carbonyl group, an amide group, a guanidyl group, a mercapto group, or the like, these groups may be protected, and a target compound can be obtained by removing the protecting groups after the reaction, as required.

Examples of the protecting group of the hydroxy group include an alkyl group, an aryl group, a trityl group, an arylalkyl group having 7 to 10 carbon atoms, a formyl group, an acyl group having 1 to 6 carbon atoms, a benzoyl group, an arylalkylcarbonyl group having 7 to 10 carbon atoms, a 2-tetrahydropyranyl group, a 2-tetrahydrofuranyl group, a silyl group, and an alkenyl group having 2 to 6 carbon atoms. These groups may be substituted with 1 to 3 substituents selected from the group consisting of a halogen atom, an alkyl group having 1 to 6 carbon atoms, an alkoxy group having 1 to 6 carbon atoms, and a nitro group.

Examples of the protecting group of the amino group include a formyl group, an acyl group having 1 to 6 carbon atoms, an alkoxycarbonyl group having 1 to 6 carbon atoms, a benzoyl group, an arylalkylcarbonyl group having 7 to 10 carbon atoms, an arylalkyloxycarbonyl group having 7 to 14 carbon atoms, a trityl group, a monomethoxytrityl group, a 1-(4,4-Dimethyl-2,6-dioxocyclohex-1-ylidene)-3-methylbutyl group, a phthaloyl group, an N,N-dimethylaminomethylene group, a silyl group, and an alkenyl group having 2 to 6 carbon atoms. These groups may be substituted with 1 to 3 substituents selected from the group consisting of a halogen atom, an alkoxy group having 1 to 6 carbon atoms, and a nitro group.

Examples of the protecting group of the carboxy group include, for example, the above-described protecting groups of the hydroxy group, a trityl group, and the like.

Examples of the protecting group of the carbonyl group include, for example, a cyclic acetal (for example, 1,3-dioxane), an acyclic acetal (for example, di(alkyl having 1 to 6 carbon atoms) acetal), and the like.

Examples of the protecting group of the amide group include a trityl group and the like.

Examples of the protecting group of the guanidyl group include a 2,2,4,6,7-pentamethyldihydrobenzofuran-5-sulfonyl group, a 2,3,4,5,6-pentamethylbenzenesulfonyl group, a tosyl group, a nitro group, and the like.

Examples of the protecting group of the mercapto group (thiol group) include a trityl group, a 4-methylbenzyl group, an acetylaminomethyl group, a t-butyl group, a t-butylthio group, and the like.

The method for removing the above-described protecting group may be performed according to a known method, for example, a method described in Protective Groups in Organic Synthesis, John Wiley and Sons (1980), or the like. Methods using an acid, a base, ultraviolet light, hydrazine, phenylhydrazine, sodium N-methyldithiocarbamate, tetrabutylammonium fluoride, palladium acetate, or a trialkylsilyl halide, and a reduction method, are used.

The term "amino acid" means an α-, β-, or γ-amino acid, and is not limited to a naturally occurring amino acid, but may also be a non-naturally occurring amino acid. In addition, the term "amino acid" may also refer to an amino acid analog such as a hydroxycarboxylic acid.

### <Compound represented by Formula (1) or Formula (2)>

In the method for producing a peptide according to the embodiment of the present invention, a compound represented by Formula (1) or Formula (2) is used.

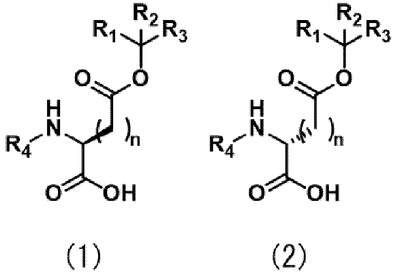

In Formula (1) and Formula (2), n is 1 or 2, R₁, R₂, and R₃ are each independently an aliphatic hydrocarbon group which may have a substituent containing no aromatic ring, at least one of R₁, R₂, and R₃ has a branched structure, two groups of R₁, R₂, and R₃ may form a ring, and R₄ represents a protecting group of an amino group.

The aliphatic hydrocarbon group may be saturated or unsaturated, and may be linear, branched, or cyclic. Here, at least one of R₁, R₂, and R₃ has a branched structure.

Preferably, at least one of R₁, R₂, and R₃ is a butyl group having a branched structure, which may have a substituent containing no aromatic ring.

The aliphatic hydrocarbon group is preferably an alkyl group or an alkenyl group, and more preferably an alkyl group.

The "alkyl group" is preferably an alkyl group having 1 to 10 carbon atoms (also referred to as "the number of carbon atoms"). The number of carbon atoms in the alkyl group is preferably 1 to 6, more preferably 1 to 4, and still more preferably 1 or 2. Specific examples thereof include methyl, ethyl, propyl, isopropyl, butyl, and tert-butyl.

An "alkenyl group" is preferably an alkenyl group having 2 to 6 carbon atoms. Specific examples thereof include 1-propenyl.

The aliphatic hydrocarbon group may have a substituent containing no aromatic ring. The aliphatic hydrocarbon group which may have a substituent containing no aromatic ring may be an aliphatic hydrocarbon group which may contain -O-.

The substituent containing no aromatic ring is not particularly limited, and examples thereof include a cycloaliphatic hydrocarbon group, a cycloaliphatic hydrocarbon group containing -O- in the ring, or an alkoxy group.

As an "alkoxy group", an alkoxy group having 1 to 10 carbon atoms is preferable. The number of carbon atoms in the alkoxy group is preferably 1 to 6, more preferably 1 to 4, and still more preferably 1 or 2. Specific examples thereof include methoxy, ethoxy, and propoxy.

Particularly preferred specific examples of the substituent containing no aromatic ring are cyclohexyl, tetrahydropyranyl, or alkoxy having 1 to 6 carbon atoms.

Examples of the protecting group of the amino group represented by R₄ include a formyl group, an acyl group having 1 to 6 carbon atoms, an alkoxycarbonyl group having 1 to 6 carbon atoms, a benzoyl group, an arylalkylcarbonyl group having 7 to 10 carbon atoms, an arylalkyloxycarbonyl group having 7 to 14 carbon atoms, a trityl group, a monomethoxytrityl group, a 1-(4,4-Dimethyl-2,6-dioxocyclohex-1-ylidene)-3-methylbutyl group, a phthaloyl group, an N,N-dimethylaminomethylene group, a silyl group, and an alkenyl group having 2 to 6 carbon atoms. These groups may be substituted with 1 to 3 substituents selected from the group consisting of a halogen atom, an alkoxy group having 1 to 6 carbon atoms, and a nitro group. Particularly preferred specific examples of the protecting group of the amino group represented by R₄ include 9-fluorenylmethyloxycarbonyl and benzyloxycarbonyl.

In Formula (1) and Formula (2), n is 1 or 2, and n is preferably 1.

Specific examples of the compound represented by Formula (1) or Formula (2) are shown below.

### L-Asp-related compounds

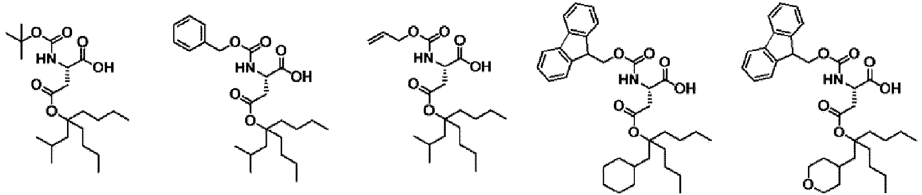

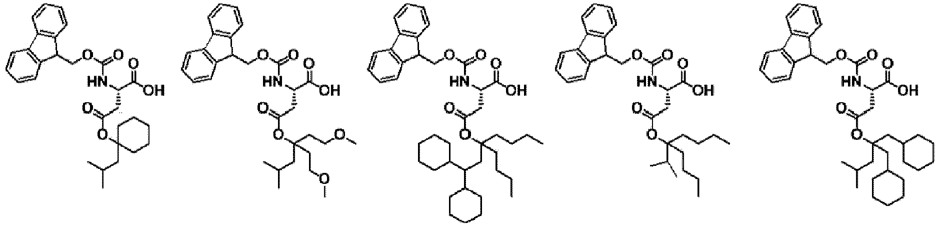

### D-Asp-related compounds

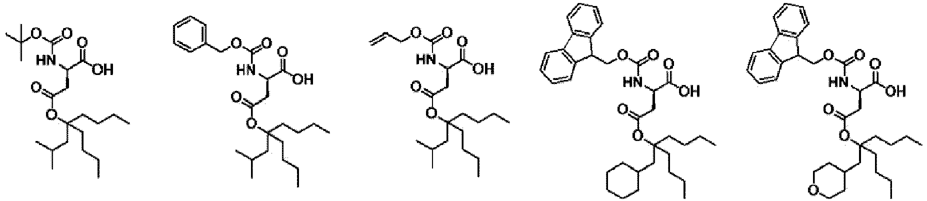

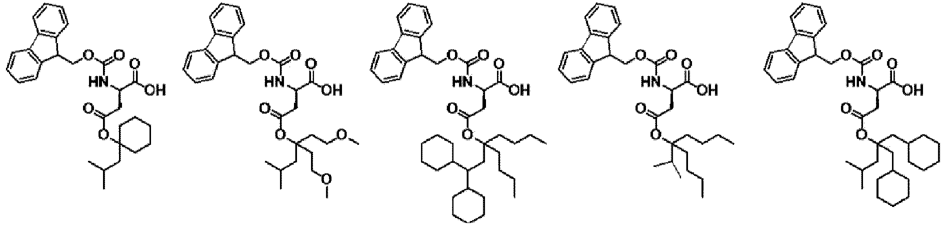

### L-Glu-related compounds

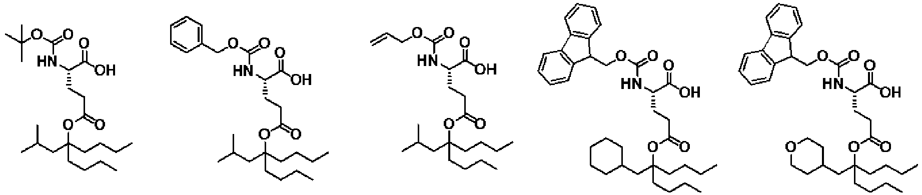

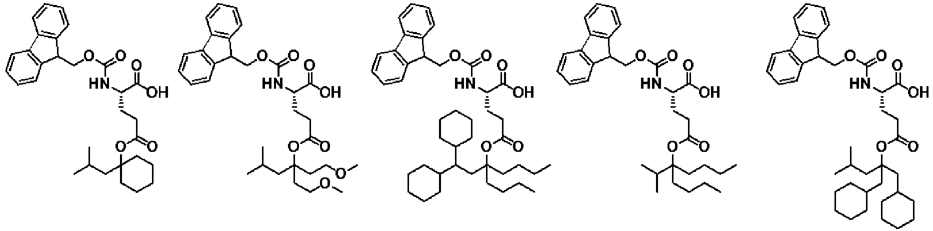

The compound represented by Formula (1) or Formula (2) can be used as a peptide synthesis reagent. The peptide synthesis will be described later.

### <Method for producing compound represented by Formula (1) or Formula (2)>

The method for producing a compound represented by Formula (1) or Formula (2) is not particularly limited, and the compound can be produced with reference to a known method.

Unless otherwise specified, a raw material compound used for producing the compound represented by Formula (1) or Formula (2) may be a commercially available compound, or may be produced according to a known method or a method analogous thereto.

In addition, the produced compound represented by Formula (1) or Formula (2) may be purified by a known purification method as necessary. For example, a method of isolating and purifying by recrystallization, column chromatography, or the like, a method of purifying by reprecipitation with a unit for changing the solution temperature, a unit for changing the solution composition, or the like, and the like can be performed.

The method of synthesizing the compound represented by Formula (1) or Formula (2) is not particularly limited, and the compound can be synthesized, for example, according to the following scheme.

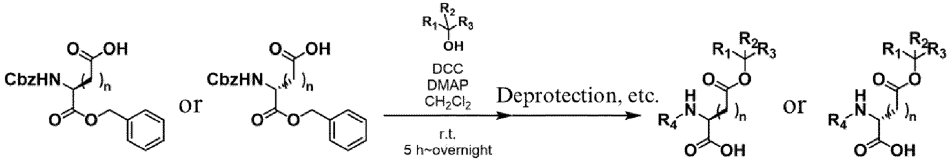

In the formula, R₁ to R₄ and n are as defined in the present specification. DCC represents N,N-dicyclohexylcarbodiimide, DMAP represents 4-dimethylaminopyridine, and r.t. represents room temperature.

### (Method for producing peptide)

The method for producing a peptide according to the present disclosure includes a step of reacting the compound represented by Formula (1) or Formula (2) with an amino terminus of an amino acid or a peptide. The amino acid or the peptide may be bonded to a solid-phase carrier such as a resin.

In the method for producing a peptide according to the present disclosure, it is preferable that the compound represented by Formula (1) or Formula (2) is an N-terminal-protected amino acid or an N-terminal-protected peptide, and
the method further includes
a peptide chain extension step of condensing the compound represented by Formula (1) or Formula (2) with a C-terminal-protected amino acid or a C-terminal-protected peptide; and
a precipitation step of precipitating an N-terminal and C-terminal-protected peptide obtained in the peptide chain extension step.

In the present invention, it is preferable that the method further includes, after the above precipitation step, the following steps one or more times in this order:
a step of deprotecting an N-terminal of the obtained N-terminal and C-terminal-protected peptide;
a step of condensing an N-terminal-protected amino acid or an N-terminal-protected peptide with the N-terminal of the obtained C-terminal-protected peptide; and
a step of precipitating the obtained N-terminal and C-terminal-protected peptide.

It should be noted that the N-terminal-protected amino acid or N-terminal-protected peptide is an amino acid or peptide in which only the N-terminal is protected, and the N-terminal and C-terminal-protected amino acid or peptide is an amino acid or peptide in which the N-terminal and the C-terminal are protected.

The formation reaction (condensation reaction) of the peptide bond between the amino group and the carboxy group, and the deprotection of the protecting group in each step can be performed by a known method. For example, WO2020/175473A is referred to, which is incorporated herein by reference.

Hereinafter, each step described above will be described in detail.

### <C-terminal protection step>

The method for producing a peptide according to the present disclosure preferably includes a C-terminal protection step of protecting a carboxy group or an amide group of the amino acid or the peptide with a C-terminal protecting group.

The C-terminal protecting group preferably has an aliphatic hydrocarbon group having 12 or more carbon atoms, and the number of carbon atoms is preferably 15 or more and more preferably 20 to 30. In a case where the C-terminal protecting group has a plurality of aliphatic hydrocarbon groups, the total number of carbon atoms therein is preferably 30 to 80 and more preferably 36 to 80. The C-terminal protecting group preferably has a ring structure, and more preferably has a condensed polycycle, an aromatic heterocyclic ring, or a naphthalene ring.

As the C-terminal protecting group, an aromatic heterocyclic compound represented by Formula (1) in WO2020/175473A is preferable. For such a compound, WO2020/175473A is referred to, which is incorporated herein by reference.

As the C-terminal protecting group, a condensed polycyclic aromatic hydrocarbon compound represented by Formula (1) in WO2020/175472A is also preferable. For such a compound, WO2020/175472A is referred to, which is incorporated herein by reference.

The C-terminal protecting group may be a compound disclosed in WO2020/262259A (JP2019-122492 and a patent application based on the same), and WO2020/262259A (JP2019-122492 and a patent application based on the same) is referred to, which is incorporated herein by reference.

The amino acid or peptide used in the above-described C-terminal protection step is not particularly limited, but is preferably an N-terminal-protected amino acid or an N-terminal-protected peptide, and more preferably an Fmoc-protected amino acid or an Fmoc-protected peptide.

In addition, it is preferable that a hydroxy group, an amino group, a carbonyl group, a carboxyl group, an amide group, an imidazole group, an indole group, a guanidyl group, a mercapto group, or the like, other than the C-terminal portion of the amino acid or peptide used in the above C-terminal protection step be protected by protecting groups.

In a case where the bonding site of the C-terminal protecting group is -OH or -SH, it is preferable to add a condensing agent in the presence of a condensation additive (condensation activating agent) in a solvent which does not affect the reaction, or to react in an acid catalyst.

In a case where the bonding site of the C-terminal protecting group is -NHR¹⁸ (R¹⁸ is a hydrogen atom, an alkyl group, an arylalkyl group, or a heteroarylalkyl group), it is preferable to add a condensing agent in the presence of a condensation additive, or to react the condensing agent with a base. As the condensation activating agent, condensing agent, and acid catalyst, WO2020/175473A is referred to, which is incorporated herein by reference.

As the condensing agent, a condensing agent generally used in peptide synthesis can be used without limitation in the present disclosure. Examples thereof include 4-(4,6-dimethoxy-1,3,5-triazin-2-yl)-4-methylmorpholinium chloride (DMT-MM), O-(benzotriazol-1-yl)-1,1,3,3-tetramethyluronium hexafluorophosphate (HBTU), O-(7-azabenzotriazol-1-yl)-1,1,3,3-tetramethyluronium hexafluorophosphate (HATU), O-(6-chlorobenzotriazol-1-yl)-1,1,3,3-tetramethyluronium hexafluorophosphate (HBTU(6-Cl)), O-(benzotriazol-1-yl)-1,1,3,3-tetramethyluronium tetrafluoroborate (TBTU), O-(6-chlorobenzotriazol-1-yl)-1,1,3,3-tetramethyluronium tetrafluoroborate (TCTU), (1-cyano-2-ethoxy-2-oxoethylidenaminooxy)dimethylamino-morpholino-carbenium hexafluorophosphate (COMU), dicyclohexylcarbodiimide (DCC), diisopropylcarbodiimide (DIC), 1-ethyl-3-(3-dimethylaminopropyl) carbodiimide (EDC), a hydrochloride salt (EDC·HCl) of 1-ethyl-3-(3-dimethylaminopropyl) carbodiimide, and (benzotriazol-1-yloxy)tripyrrolidinophosphonium hexafluorophosphate (PyBoP), but the condensing agent is not limited thereto.

Among these, DIC, EDC, EDC·HCl, DMT-MM, HBTU, HATU, or COMU is preferable.

An amount of the condensing agent to be used is preferably 1 molar equivalent to 10 molar equivalents and more preferably 1 molar equivalent to 5 molar equivalents with respect to 1 molar equivalent of the substrate.

As the acid catalyst used in the condensation reaction, an acid catalyst generally used in peptide synthesis can be used without limitation, and examples thereof include methanesulfonic acid, trifluoromethanesulfonic acid, and p-toluenesulfonic acid.

Among these, methanesulfonic acid or p-toluenesulfonic acid is preferable.

An amount of the acid catalyst to be used is preferably 1 molar equivalent to 10 molar equivalents and more preferably 1 molar equivalent to 5 molar equivalents with respect to 1 molar equivalent of the substrate.

In the above-described C-Terminal Protection Step, it is preferable to add the condensation activating agent in order to promote the reaction and suppress side reactions such as racemization.

The condensation activating agent is a reagent which, in a case of coexisting with the condensing agent, leads an amino acid to a corresponding active ester, acid anhydride, or the like to facilitate the formation of a peptide bond (amide bond).

As the condensation activating agent, an activating agent generally used in peptide synthesis can be used without limitation, and examples thereof include 4-dimethylaminopyridine, N-methylimidazole, boronic acid derivative, 1-hydroxybenzotriazole (HOBt), ethyl 1-hydroxytriazole-4-carboxylate (HOCt), 1-hydroxy-7-azabenzotriazole (HOAt), 3-hydroxy-1,2,3-benzotriazin-4(3H)-one (HOOBt), N-hydroxysuccinimide (HOSu), N-hydroxyphthalimide (HOPht), N-hydroxy-5-norbornene-2,3-dicarboxyimide (HONb), pentafluorophenol, and ethyl (hydroxyimino)cyanoacetate (Oxyma). Among these, 4-dimethylaminopyridine, HOBt, HOCt, HOAt, HOOBt, HOSu, HONb, or Oxyma is preferable.

An amount of the activating agent to be used is preferably more than 0 molar equivalent to 4.0 molar equivalents and more preferably 0.1 molar equivalent to 1.5 molar equivalents with respect to 1 molar equivalent of the substrate.

As the solvent, a general organic solvent can be used in the reaction. Specific examples thereof include halogenated hydrocarbons such as chloroform and dichloromethane; and nonpolar organic solvents such as 1,4-dioxane, tetrahydrofuran (THF), and cyclopentyl methyl ether. Two or more kinds of these solvents may be mixed and used. In addition, aromatic hydrocarbons such as benzene, toluene, and xylene; nitriles such as acetonitrile and propionitrile; ketones such as acetone and 2-butanone; amides such as N,N-dimethylformamide and N-methylpyrrolidone; and sulfoxides such as dimethyl sulfoxide may be used by being mixed with the above-described halogenated hydrocarbons or nonpolar organic solvents.

The reaction temperature is not particularly limited, but is preferably -10°C to 80°C and more preferably 0°C to 40°C. The reaction time is not particularly limited, but is preferably 1 hour to 30 hours.

In addition, the N-terminal and C-terminal-protected amino acid or N-terminal and C-terminal-protected peptide obtained by the above-described C-Terminal Protection Step may be purified.

For example, the product obtained after dissolving the obtained N-terminal and C-terminal-protected amino acid compound or N-terminal and C-terminal-protected peptide in a solvent (reaction solvent, for example, THF) and performing a desired organic synthesis reaction is isolated. Thereafter, the solvent in which the N-terminal and C-terminal-protected amino acid compound or N-terminal and C-terminal-protected peptide is dissolved is changed (for example, by changing the solvent composition or the type of solvent), thereby causing reprecipitation.

Specifically, the reaction is performed under conditions such that the N-terminal and C-terminal-protected amino acid or N-terminal and C-terminal-protected peptide is dissolved. After the reaction, the solvent is distilled off and then replaced with another solvent, or a polar solvent is added to the reaction system without distilling off the solvent, thereby precipitating aggregates and removing impurities.

As the replacement solvent or the polar solvent, polar organic solvents such as methanol, acetonitrile, and water are used alone or in combination. That is, the reaction is performed under conditions such that the N-terminal and C-terminal-protected amino acid or N-terminal and C-terminal-protected peptide is dissolved, and after the reaction, as the solvent replacement, for example, a halogenated solvent, THF, or the like is used for dissolution, and a polar organic solvent such as methanol, acetonitrile, and water is used for precipitation.

### <N-terminal deprotection step>

The method for producing a peptide according to the present disclosure preferably includes an N-terminal deprotection step of deprotecting an N-terminal protecting group of the N-terminal and C-terminal-protected amino acid or N-terminal and C-terminal-protected peptide obtained in the above-described C-terminal protection step.

As the N-terminal protecting group, a protecting group for an amino group described later, which is generally used in a technical field such as peptide chemistry, can be used. In the present disclosure, a Boc group, a Cbz group, or an Fmoc group is suitable.

The deprotection conditions are appropriately selected depending on the type of the temporary protecting group. For example, in a case of the Fmoc group, the deprotection is performed by treating with a base, and in a case of the Boc group, the deprotection is performed by treating with an acid. The reaction is performed in a solvent which does not affect the reaction.

Examples of the base include secondary amines such as dimethylamine and diethylamine, and non-nucleophilic organic bases such as 1,8-diazabicyclo[5.4.0]-7-undecene (DBU), 1,4-diazabicyclo[2.2.2]octane (DABCO), and 1,5-diazabicyclo[4.3.0]-5-nonene (DBN).

As the solvent, the solvent described above for the <C-terminal protection step> may suitably be used.

### <Peptide chain extension step>

The method for producing a peptide according to the present disclosure preferably includes a peptide chain extension step of condensing an N-terminal-protected amino acid or an N-terminal-protected peptide to the N-terminal of the C-terminal-protected amino acid or C-terminal-protected peptide obtained in the above-described N-terminal deprotection step.

The above-described peptide chain extension step is suitably performed under peptide synthesis conditions generally used in the field of peptide chemistry, in which the above-described condensing agent, condensation additive, and the like are used.

The N-terminal-protected amino acid or the N-terminal-protected peptide is not particularly limited, and an Fmoc-protected amino acid or an Fmoc-protected peptide is suitable.

### <Precipitation step>

It is preferable that the method for producing a peptide according to the present disclosure further includes a precipitation step of precipitating the N-terminal and C-terminal-protected peptide obtained in the above-described peptide chain extension step.

The precipitation step can be performed in the same manner as in the purification (reprecipitation) of the C-terminal protection step described above.

Specifically, a polar solvent is added to the reaction system without distilling off the reaction solvent after the reaction in the previous stage. In this case, in the reaction solvent, THF is used as the nonpolar organic solvent and acetonitrile is used as the polar solvent. The proportion (volume basis) of the nonpolar organic solvent and the polar solvent used is preferably 1:1 to 1:100, more preferably 1:3 to 1:50, and still more preferably 1:5 to 1:20. In the case of this proportion used, the N-terminal and C-terminal-protected amino acid compound or N-terminal and C-terminal-protected peptide can be efficiently precipitated, and the target product can be efficiently purified.

### <Chain extension>

It is preferable that the method for producing a peptide according to the present disclosure further includes, after the above-described precipitation step, the following steps one or more times in this order, a step of deprotecting the N-terminal of the obtained N-terminal and C-terminal-protected peptide, a step of condensing the N-terminal of the obtained C-terminal-protected peptide with an N-terminal-protected amino acid or an N-terminal-protected peptide, and a step of precipitating the obtained N-terminal and C-terminal-protected peptide.

By repeating the above-described three steps, the chain extension of the obtained peptide can be easily performed.

Each step in the above-described three steps can be performed in the same manner as each corresponding step described above.

### <C-terminal deprotection step>

It is preferable that the method for producing a peptide according to the present disclosure further includes a C-terminal deprotection step of deprotecting a C-terminal protecting group.

In the above-described C-terminal deprotection step, by removing the above-described C-terminal protecting group in the C-terminal-protected peptide having a desired number of amino acid residues, a peptide as a final target product can be obtained.

Preferred examples of a method of removing the C-terminal protecting group include a deprotecting method using an acidic compound.

Examples thereof include a method of adding an acid catalyst and a hydrogenating method using a metal catalyst. Examples of the acid catalyst include trifluoroacetic acid (TFA), hydrochloric acid, trifluoroethanol (TFE), hexafluoroisopropanol (HFIP), and acetic acid. TFA is preferable for peptides which do not decompose with strong acids, and TFE, HFIP, or acetic acid is preferable for peptides which decompose with strong acids. A concentration of the acid can be appropriately selected depending on the side chain protecting group of the extending amino acid and the deprotection conditions.

For example, a concentration of TFA is preferably 50% by volume or less, more preferably 30% by volume or less, still more preferably 10% by volume or less, even more preferably 5% by volume or less, and particularly preferably 1% by volume or less with respect to the total volume of the solvent used. The lower limit value thereof is preferably 0.01% by volume, more preferably 0.1% by volume, and still more preferably 0.5% by volume.

The deprotection time is preferably 5 hours or less, more preferably 3 hours or less, and still more preferably 1 hour or less.

The peptide, which is the final target product obtained by the method for producing a peptide according to the present disclosure, can be isolated and purified according to a method commonly used in peptide chemistry. For example, the peptide, which is the final target product, can be isolated and purified by extraction washing the reaction mixture, crystallization, chromatography, and the like.

The type of peptide produced by the method for producing a peptide according to the present disclosure is not particularly limited, but it is preferable that the number of amino acid residues of the peptide is, for example, approximately several tens or less. Similar to existing or unknown synthetic or native peptides, the peptide obtained by the method for producing a peptide according to the present disclosure can be used in various fields such as pharmaceuticals, foods, cosmetics, electronic materials, biosensors, and the like, but the use of the peptide is not limited thereto.

In the method for producing a peptide according to present disclosure, the precipitation step can be appropriately omitted as long as it does not affect the reaction in the next step.

### Examples

Hereinafter, the embodiments of the present invention will be described more specifically with reference to Examples. The materials, amounts to be used, proportions, treatment contents, treatment procedures, and the like shown in Examples can be appropriately modified as long as the modifications do not depart from the spirit of the embodiments of the present invention. Therefore, it is to be understood that the scope of the embodiments of the present invention is not limited to the following specific examples. Unless otherwise specified, "%" is based on mass. The room temperature means 25°C.

Unless otherwise specified, purification by column chromatography was performed using an automatic purification device ISOLERA (manufactured by Biotage Ltd.) or a medium pressure liquid chromatograph YFLC-Wprep 2XY.N (manufactured by YAMAZEN).

Unless otherwise specified, SNAPKP-Sil Cartridge (manufactured by Biotage Ltd.) or a high flash column WO01, WO02, WO03, WO04, or WO05 (manufactured by YAMAZEN) was used as a carrier in silica gel column chromatography.

The mixing ratio in an eluant used for column chromatography is the volume ratio. For example, "gradient elution of hexane:ethyl acetate = 50:50 to 0:100" means that an eluant of 50% hexane and 50% ethyl acetate is finally changed to an eluant of 0% hexane and 100% ethyl acetate.

In addition, "gradient elution of hexane:ethyl acetate = 50:50 to 0:100 and gradient elution of methanol:ethyl acetate = 0:100 to 20:80" means that an eluant of 50% hexane and 50% ethyl acetate is changed to an eluant of 0% hexane and 100% ethyl acetate, and then the eluant of 0% hexane and 100% ethyl acetate is finally changed to an eluant of 20% methanol and 80% ethyl acetate.

MS spectrum was measured using ACQUITY SQD LC/MS System (manufactured by Waters Corporation; ionization method: electrospray ionization (ESI) method).

The HPLC purity was measured using ACQUITY UPLC (manufactured by Waters Corporation, column: CSH C18 1.7 µm).

The following compounds were used. A compound purchased from Tokyo Chemical Industry Co., Ltd. (catalog number: B3150) was used as the compound of Comparative Example 1, and a compound purchased from Sigma-Aldrich (catalog number: 8.52418) was used as the compound of Comparative Example 2.

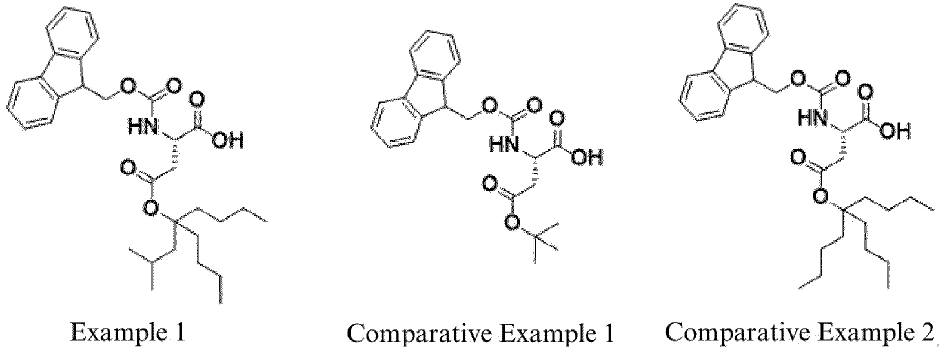

### <Synthesis of compound of Example 1>

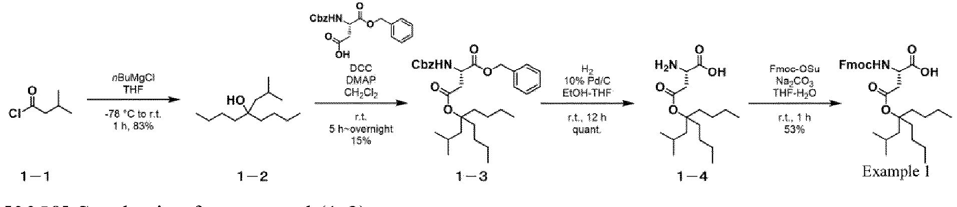

### Synthesis of compound (1-2)

2.03 g of isovaleryl chloride (compound 1-1, manufactured by Tokyo Chemical Industry Co., Ltd.) and 40 mL of tetrahydrofuran (ultra-dehydrated grade, manufactured by FUJIFILM Wako Pure Chemical Corporation) were placed in a 200 mL three-neck round-bottom flask, an n-butyl lithium hexane solution (1.6 mol/L, manufactured by Kanto Chemical Co., Inc.) was added dropwise thereto at -78°C, and the mixture was stirred at room temperature for 1 hour. After completion of the reaction, a saturated ammonium chloride aqueous solution was added thereto, and an extraction operation was performed with ethyl acetate. The obtained organic layer was washed with saturated saline, dried using sodium sulfate, and then filtered to remove insoluble matter. The residue obtained by concentrating the filtrate under reduced pressure was purified by silica gel column chromatography (hexane/ethyl acetate = 10/1) and dried to obtain 2.78 g of a compound (1-2) as a colorless transparent syrupy substance.

### Synthesis of compound (1-3)

800 mg of the compound (1-2), 500 mg of N-benzyloxycarbonyl-L-aspartic acid 4-benzyl (manufactured by Tokyo Chemical Industry Co., Ltd.), 34 mg of 4-dimethylaminopyridine (manufactured by Tokyo Chemical Industry Co., Ltd.), and 10 mL of dichloromethane (manufactured by FUJIFILM Wako Pure Chemical Corporation) were placed in a 50 mL round-bottom flask, and 1.17 g of N,N-dicyclohexylcarbodiimide (manufactured by FUJIFILM Wako Pure Chemical Corporation) was added thereto at room temperature in four portions at one-hour intervals. After further stirring at room temperature for 1 hour, 247 µL of acetic acid was added thereto. After filtering off insoluble matter, the residue obtained by concentrating the filtrate under reduced pressure was purified by silica gel column chromatography (hexane/ethyl acetate = 10/1) and dried to obtain 111 mg of a compound (1-3) as a colorless transparent syrupy substance.

### Mass of the obtained compound (1-3): [M + Na] 563

### Synthesis of compound (1-4)

111 mg of the compound (1-3), 28 mg of 10% palladium-activated carbon (approximately 55% water-wet product, manufactured by FUJIFILM Wako Pure Chemical Corporation), 1.0 mL of tetrahydrofuran (manufactured by FUJIFILM Wako Pure Chemical Corporation), and 1.2 mL of ethanol (manufactured by FUJIFILM Wako Pure Chemical Corporation) were placed in a 50 mL pressure-resistant reaction container, and the mixture was stirred at room temperature for 10 hours in a hydrogen atmosphere. After completion of the reaction, insoluble matter was filtered off by a filtration operation using celite. The obtained filtrate was concentrated under reduced pressure and dried to obtain 65 mg of a compound (1-4) as a white solid.

### Mass of the obtained compound (1-4): [M - H] 314

### Synthesis of Example (1)

65 mg of the compound (1-4), 90 mg of sodium carbonate (manufactured by FUJIFILM Wako Pure Chemical Corporation), 1.3 mL of tetrahydrofuran (manufactured by FUJIFILM Wako Pure Chemical Corporation), and 1.3 mL of distilled water were placed in a 50 mL round-bottom flask, 105 mg of N-[(9H-fluoren-9-ylmethoxy)carbonyloxy]succinimide (manufactured by FUJIFILM Wako Pure Chemical Corporation) was added thereto at room temperature, and the mixture was stirred for 1 hour. After completion of the reaction, a saturated ammonium chloride aqueous solution and chloroform were added thereto, the organic layer was separated, and then the organic layer was dried using sodium sulfate. Insoluble matter was filtered off, and the residue obtained by concentrating the filtrate under reduced pressure was purified by silica gel column chromatography (chloroform/methanol = 10/1) and dried to obtain 59 mg of a compound of Example 1 as a white amorphous solid.

### Mass of the obtained compound of Example 1: [M + Na] 561

### <Comparative data>

The aspartimide (Asi) formation rate and the target product purity in a case where H₂N-Asp-Gly-Gly-Phe-OH was synthesized using, as the compound, the compound used in the Example, Comparative Example 1, or Comparative Example 2 and using H₂N-Asp-Gly-Gly-Phe-OH as the model sequence were obtained by calculating area values in ultra-performance liquid chromatography (UPLC). For the synthesis of the model sequence, a method of introducing a substituted benzyl compound described in WO2023/106356A into the C-terminal can be used.

Aspartimide (Asi) formation rate
Evaluation A: the Asi formation rate was less than 1%
Evaluation B: the Asi formation rate was 1% or more and less than 3%
Evaluation C: the Asi formation rate was 3% or more and less than 5%
Evaluation D: the Asi formation rate was 5% or more

Target product purity
Evaluation A: the target product purity was 95% or more
Evaluation B: the target product purity was 93% or more and less than 95%
Evaluation C: the target product purity was 90% or more and less than 93%
Evaluation D: the target product purity was less than 90%

The results of evaluating the aspartimide (Asi) formation rate and the target product purity are shown in the following table.

**[Table 1]**

| Compound | Asi formation rate (%) | Target product purity (%) |
|---|---|---|
| Example 1 | A | A |
| Comparative Example 1 | D | D |
| Comparative Example 2 | C | C |

### <Synthesis of protected peptide (4-residue peptide: H₂N-Asp(X)-Gly-Gly-Phe-OH)>

Details of each abbreviation other than the above are shown below.
Phe: phenylalanine residue
Gly: glycine residue
Asp(X): asparagine residue having a protecting group according to the embodiment of the present invention

### (Synthesis of Fmoc-Phe-O-TAG)

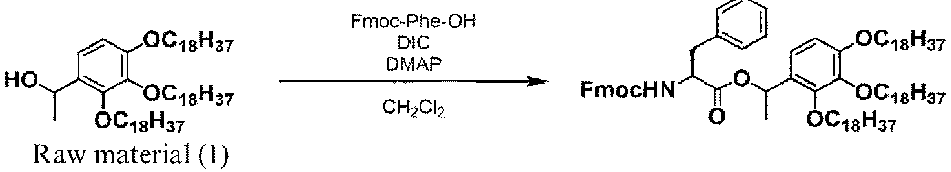

A raw material (1) (10.0 g, 10.78 mmol) synthesized according to Examples of WO2023/106356A was dissolved in dichloromethane (110 mL), and Fmoc-Phe-OH (1.5 molar equivalents), 4-dimethylaminopyridine (0.2 molar equivalent), and diisopropylcarbodiimide (1.5 molar equivalents) were added thereto and stirred. After the completion of the condensation reaction, an 80% methanol aqueous solution (550 mL) was added thereto and stirred, and the precipitate was collected by filtration and dried under reduced pressure to obtain Fmoc-Phe-O-TAG (14.0 g, yield: 100%).

### (Synthesis of H₂N-Asp(X)-Gly-Gly-Phe-O-TAG)

Using the Fmoc-Phe-O-TAG, a peptide sequence was extended by repeating the removal of the Fmoc group and the condensation reaction of the protected amino acid shown in Table 2.

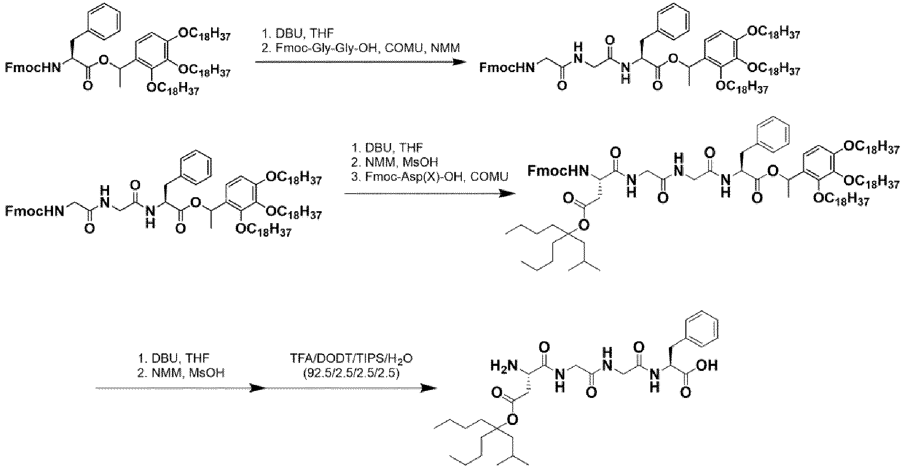

**[Table 2]**

| Protected amino acid | Sequence | Yield [%] |
|---|---|---|
| Fmoc-Gly-Gly-OH | Fmoc-Gly-Gly-Phe-O-TAG | 96.7 |
| Fmoc-Asp(X)-OH | Fmoc-Asp(X)-Gly-Gly-Phe-O-TAG | 98.3 |
| Fmoc deprotection | H₂N-Asp(X)-Gly-Gly-Phe-O-TAG | 92.7 |

### (Deprotection of peptide)

Trifluoroacetic acid/3,6-dioxa-1,8-octanedithiol/triisopropylsilane/water (92.5/2.5/2.5/2.5: vol%, 10 mL) was added to H₂N-Asp(X)-Gly-Gly-Phe-O-TAG (384 mg) at room temperature, and the mixture was stirred at room temperature for 1 hour. The reaction solution was added dropwise to ice-cooled cyclopropyl methyl ether (50 mL) to precipitate, and the supernatant was removed and the precipitate was washed with cyclopropyl methyl ether repeatedly to obtain H₂N-Asp(X)-Gly-Gly-Phe-OH (102 mg).
HPLC purity (220 nm): 95%
MS (ESI, m/z): 395 (M + H)

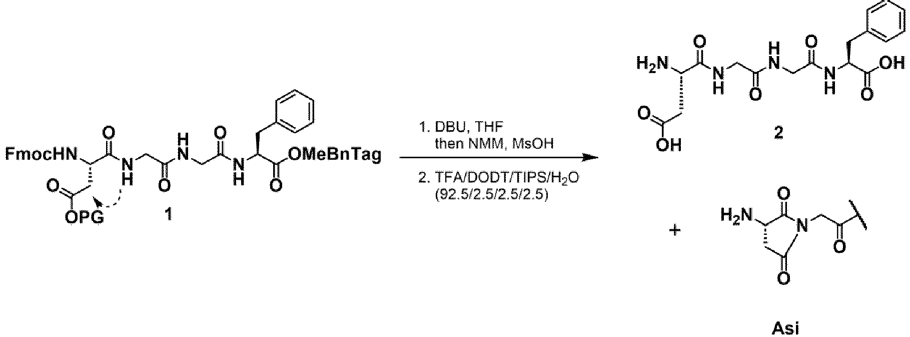

## Claims

1. A method for producing a peptide, comprising:
a step of reacting a compound represented by Formula (1) or Formula (2) with an amino terminus of an amino acid or a peptide, 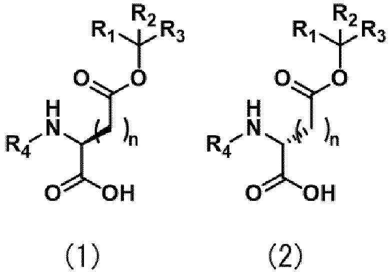
in Formula (1) and Formula (2), n is 1 or 2, R₁, R₂, and R₃ are each independently an aliphatic hydrocarbon group which may have a substituent containing no aromatic ring, at least one of R₁, R₂, and R₃ has a branched structure, two groups of R₁, R₂, and R₃ may form a ring, and R₄ represents a protecting group of an amino group.

2. The method for producing a peptide according to claim 1,
wherein the compound represented by Formula (1) or Formula (2) is an N-terminal-protected amino acid or an N-terminal-protected peptide, and
the method further comprises
a peptide chain extension step of condensing the compound represented by Formula (1) or Formula (2) with a C-terminal-protected amino acid or a C-terminal-protected peptide; and
a precipitation step of precipitating an N-terminal and C-terminal-protected peptide obtained in the peptide chain extension step.

3. The method for producing a peptide according to claim 2, further comprising, after the precipitation step, the following steps one or more times in this order:
a step of deprotecting an N-terminal of the obtained N-terminal and C-terminal-protected peptide;
a step of condensing the N-terminal-protected amino acid or the N-terminal-protected peptide with the N-terminal of the obtained C-terminal-protected peptide; and
a step of precipitating the obtained N-terminal and C-terminal-protected peptide.

4. The method for producing a peptide according to claim 2, further comprising:
a C-terminal deprotection step of deprotecting a C-terminal protecting group,
wherein the deprotection is performed using a trifluoroacetic acid solution of 10% by volume or less.

5. The method for producing a peptide according to claim 2,
wherein a C-terminal protecting group of the C-terminal-protected amino acid or the C-terminal-protected peptide has an aliphatic hydrocarbon group having 12 or more carbon atoms.

6. The method for producing a peptide according to any one of claims 1 to 5,
wherein the aliphatic hydrocarbon group which may have a substituent containing no aromatic ring is an aliphatic hydrocarbon group which may contain -O-.

7. The method for producing a peptide according to any one of claims 1 to 5,
wherein the substituent containing no aromatic ring is a cycloaliphatic hydrocarbon group, a cycloaliphatic hydrocarbon group containing -O- in a ring, or an alkoxy group.

8. The method for producing a peptide according to any one of claims 1 to 5,
wherein the substituent containing no aromatic ring is cyclohexyl, tetrahydropyranyl, or alkoxy having 1 to 6 carbon atoms.

9. The method for producing a peptide according to any one of claims 1 to 5,
wherein R₄ is 9-fluorenylmethyloxycarbonyl or benzyloxycarbonyl.

10. The method for producing a peptide according to any one of claims 1 to 5,
wherein at least one of R₁, R₂, and R₃ is a butyl group having the branched structure, which may have the substituent containing no aromatic ring.

11. The method for producing a peptide according to any one of claims 1 to 5,
wherein n is 1.

12. A compound represented by Formula (1) or Formula (2), 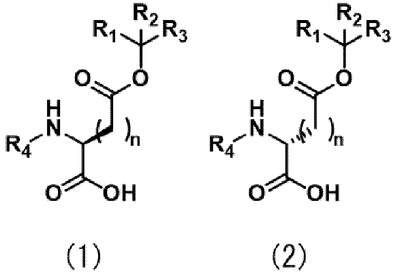 in Formula (1) and Formula (2), n is 1 or 2, R₁, R₂, and R₃ are each independently an aliphatic hydrocarbon group which may have a substituent containing no aromatic ring, at least one of R₁, R₂, and R₃ has a branched structure, two groups of R₁, R₂, and R₃ may form a ring, and R₄ represents a protecting group of an amino group.

13. The compound according to claim 12,
wherein the aliphatic hydrocarbon group which may have the substituent containing no aromatic ring is an aliphatic hydrocarbon group which may contain -O-.

14. The compound according to claim 12,
wherein the substituent containing no aromatic ring is a cycloaliphatic hydrocarbon group, a cycloaliphatic hydrocarbon group containing -O- in a ring, or an alkoxy group.

15. The compound according to claim 12,
wherein the substituent containing no aromatic ring is cyclohexyl, tetrahydropyranyl, or alkoxy having 1 to 6 carbon atoms.

16. The compound according to any one of claims 12 to 15,
wherein R₄ is 9-fluorenylmethyloxycarbonyl or benzyloxycarbonyl.

17. The compound according to any one of claims 12 to 15,
wherein at least one of R₁, R₂, and R₃ is a butyl group having the branched structure, which may have the substituent containing no aromatic ring.

18. The compound according to any one of claims 12 to 15,
wherein n is 1.

19. A peptide synthesis reagent comprising:
the compound according to any one of claims 12 to 15.
